(19) Europäisches Patentamt
European Patent Office
Office européen des brevets

(11) **EP 2 968 682 B1**

(12) **EUROPEAN PATENT SPECIFICATION**

(45) Date of publication and mention
of the grant of the patent:
**14.08.2019 Bulletin 2019/33**

(51) Int Cl.:
*A61L 29/04* *(2006.01)*        *A61L 29/10* *(2006.01)*
*A61L 29/18* *(2006.01)*

(21) Application number: **14770578.4**

(86) International application number:
**PCT/US2014/026182**

(22) Date of filing: **13.03.2014**

(87) International publication number:
**WO 2014/151657 (25.09.2014 Gazette 2014/39)**

(54) **ALCOHOL RESISTANT CATHETERS AND USES THEREOF**

ALKOHOLRESISTENTE KATHETER UND VERWENDUNGEN DAVON

CATHÉTERS RÉSISTANT À L'ALCOOL ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2013 US 201361793116 P**

(43) Date of publication of application:
**20.01.2016 Bulletin 2016/03**

(73) Proprietor: **Bard Access Systems, Inc.
Salt Lake City, UT 84116 (US)**

(72) Inventors:
• **MUSE, Jay A.
Salt Lake City, Utah 84105 (US)**
• **SESSION, Travis
Salt Lake City, Utah 84119 (US)**

• **DRAPER, Matt
North Salt Lake, Utah 84054 (US)**
• **PATTERSON, Ryan
Farmington, Utah 84025 (US)**

(74) Representative: **Bradley, Adrian et al
Cleveland LLP
10 Fetter Lane
London EC4A 1BR (GB)**

(56) References cited:
**EP-A2- 2 248 542        WO-A1-99/24084
WO-A2-2007/089724        WO-A2-2008/024514
US-A1- 2007 078 437        US-A1- 2007 078 437
US-A1- 2011 172 644        US-A1- 2011 172 644
US-A1- 2012 116 323**

EP 2 968 682 B1

## Description

<u>Technical Field</u>

The current disclosure relates generally to alcohol resistant polymers for use in medical applications. More specifically, the current disclosure relates to alcohol resistant aromatic polycarbonate urethanes for use in catheters.

<u>Description of prior art</u>

EP2248542 describes a vascular catheter comprising a catheter shaft having one or more lumens. The catheter shaft comprises a polycarbonate polyurethane and bismuth oxychloride.
US2007/007437 a catheter including a multiple lumen catheter tube, a hub attached to the catheter tube, and a first extension leg and a second extension leg attached to the hub.
WO2008/024514 describes a central venous access device comprising an extruded catheter shaft having one or more lumens, said catheter shaft comprising a polycarbonate polyurethane having a weight average molecule weight in excess of 90,000.

<u>Description of the Figures</u>

Figure 1 is a representation of a peripherally inserted central line catheter (PICC) according to certain embodiments of the disclosure.
Figure 2 is a graph showing the hourly effect of alcohol on the burst pressure of an embodiment of a catheter produced as disclosed herein.
Figure 3 is a graph showing the daily effect of alcohol on the burst pressure of an embodiment of a catheter produced as disclosed herein.
Figure 4 is a graph showing the effect of alcohol on the modulus of elasticity of some embodiments of alcohol resistant aromatic polycarbonate urethane formulations as disclosed herein.
Figure 5 is a graph showing the effect of alcohol on the tensile strength, in pounds force (Ibf), of some embodiments of alcohol resistant aromatic polycarbonate urethane formulations as disclosed herein.

<u>Detailed Description</u>

*1. Definitions*

As used herein, "medical catheter" or "catheter" refers to a medical device that includes a flexible shaft, which contains one or more lumens which may be inserted into a subject for introduction of material (e.g., fluids, nutrients, medications, blood products, etc.), monitoring of the subject (e.g., pressure, temperature, fluid); and more removal of material (e.g., body fluids), or any combination thereof. A catheter may further include various accessory components such as extension tubes, fittings, over molded junction hub, and so forth. A catheter may also have various tip and shaft features including holes, splits, tapers, overmolded tips or bumps, and so forth. A catheter may also have various tip and shaft features including holes, splits, tapers, overmolded tips or bumps, and so forth.
As used herein, "venous access device" refers to a device that provides access to the venous circulation, typically the central venous circulation system. This includes but is not limited to central venous catheters, peripherally inserted venous catheters, midlines, ports, and dialysis catheters. Venous access devices may remain in place from days to years. The typical construction of a venous access catheter includes a flexible shaft with one or multiple lumens with various tips, splits, tapers, and so forth, that is connected by a junction hub to extension tubes with luer fitting for attachment to other devices.
As used herein, "central venous catheter" refers to a catheter with its tip placed directly in the central venous circulation system. These include any device, whether wholly implanted or partially implanted that delivers medication to the central parts of the heart, such as the vena cava.
As used in this specification and the appended claims, the singular forms "a," "an," and, "the" include plural referents unless the context clearly dictates otherwise. Thus, for example, reference to "a device" may include one or more of such devices, reference to "a diol" may include reference to one or more diols, and reference to "an aromatic polycarbonate urethane" may include reference to one or more of such compounds.
As used herein, "urethane linkage" refers to the -HN-(C=O)O- moiety along the backbone of a polymer.
As used herein, "carbonate linkage" refers to the -O(C=O)O- moiety along the backbone of a polymer.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 micron to about 5 microns" should be interpreted to include not only the explicitly recited values of about 1 micron to about 5 microns, but also include individual values and sub-ranges within the indicated range. Thus, included in this numerical range are individual values such as 2, 3.5, and 4 and sub-ranges such as from 1-3, from 2-4, and from 3-5, etc.

This same principle applies to ranges reciting only one numerical value. For example, a range of values designated as less than 5, includes ranges less than 4 and less than 3. Furthermore, such an interpretation should apply regardless of the breadth of the range or the characteristics being described.

## II. Alcohol Resistant Catheters

In current medical practice, it is commonly necessary to introduce a catheter into the central venous circulation system for various purposes. For example, catheters may be introduced for purposes of delivering fluids, nutrition, blood, glucose solutions, medications, diagnostic agents, and so forth, to the vasculature. Catheters may also be introduced for the purposes of withdrawing blood from the vasculature, for example, in order to treat the blood, to carry out diagnostics on the blood, and so forth. In the process of carrying out such medically necessary tasks, a central catheter can become colonized with microbes, such as bacterial and fungus, that can harm the patient. Additionally, in the case of the delivery of nutrition, the catheter can become occluded with lipids.

A method of reducing or eliminating said microbes or said lipid occlusion is through the direct and prolonged exposure of the central access device to an alcohol, such as ethanol. One such method of exposing the central catheter to alcohol is referred to by clinicians as an alcohol lock. Alcohol locking of a central catheter refers generally to techniques or procedures where alcohol is introduced into the catheter lumen and maintained in the lumen for a period of time greater than 10 minutes with an ethanol concentration from between 25% and 100%, for the purpose of disinfection or lipid occlusion removal. The practice of alcohol locking, or the internal or external application of liquid alcohol, is referred to as direct and prolonged exposure.

Silicone catheters are generally used as central catheters when there will be direct and prolonged exposure to alcohol. It is well known by clinicians and manufacturers that direct and prolonged exposure to alcohol can adversely affect the material properties of polyurethane catheters. When direct and prolonged exposure to alcohol is not used, polyurethane catheters are often used by clinicians over silicone catheters for increased durability, particularly in power injection applications requiring high flow rates and associated high pressures.

Direct and prolonged exposure of alcohol to central access devices manufactured with polyurethanes such as Tecoflex, Quadrathane, Quadraflex, Tecothane, Pellethane, Chronoflex and the like, results in the loss of the current standard of performance, such as burst during power injection or leak due to cyclic kink. This loss of performance is directly related to alcohol-related degradation in mechanical properties such as increased swell, decreased stress crack resistance, and loss of certain mechanical properties such as hardness, modulus, and strength. Accordingly, manufacturers of central venous catheters, in some instances, explicitly disallow the use of direct and prolonged exposure to alcohol with their catheters.

The catheter shafts for central venous catheters are typically made from polymers. Suitable polymers are those that are biocompatible, that can be formed into tubing, and that are flexible enough to be routed through the vasculature without causing trauma to the patient. When formed into tubing, the polymer chosen should also provide strength sufficient to ensure that the lumen does not collapse in the vasculature, and should resist repeated flexure. Silicone and polyurethane based polymers are commonly employed to meet these criteria, however polyurethane catheters may be preferred because they are stronger.

Furthermore, catheter shafts and accessories may be made from biocompatible flexible polymers that enable them to be inserted into the body and vasculature while causing minimal trauma to the patient. These materials may generally be required to provide chemical resistance, flexibility, biocompatibility, softness, strength, burst resistance, radiopacity, and durability. In such embodiments, some catheters may be formed of thermoplastic polyurethanes. Thermoplastic polyurethanes may be melt processable and may be extruded and/or molded using heat processing, while thermoset polyurethanes may be cast molded.

In some cases, thermoplastic polyurethanes, including aliphatic and aromatic polycarbonate polyurethanes, can be subject to swelling in the presences of alcohol, water, and strong polar solvents. For example, urethanes and resultant central venous catheters when exposed to these agents may soften, swell, and lose their mechanical properties, such as modulus of elasticity and tensile strength. This effect may also be accelerated at body temperatures. The resultant loss of these mechanical properties may cause central venous catheter failures including, but not limited to tip instability, tip malposition, bursts during power injection, lumen collapse during fluid aspiration, cyclic fatigue failures from repeated bending, and leakage at the junction hub from the extension legs or the catheter shaft. Accordingly, in many applications, medical device manufacturers are required to design-in safety factors, specify the conditions under which polyurethane central venous catheters may be used, and disallow the use of alcohol and other materials with the catheters to prevent these failures.

The present disclosure relates to alcohol resistant aromatic polycarbonate urethane polymers. Also disclosed herein are alcohol resistant catheters comprising the alcohol resistant aromatic polycarbonate urethane polymers disclosed herein. The alcohol resistant aromatic polycarbonate urethane is formed by reacting at least the following monomers: a polyisocyanate, a polyol, and a chain extender. The monomers provide an aromatic polycarbonate urethane having reduced swelling, improved stress crack resistance, and/or greater retention of certain mechanical properties such as hardness, modulus, and strength, upon exposure to alcohol. The monomers provide an aromatic polycarbonate urethane having a plurality of urethane linkages and a plurality of carbonate linkages and where the aromatic polycarbonate (relative to certain polyurethanes such as Tecoflex, Quadrathane, Quadraflex, Tecothane, Pellethane, Chronoflex and the like) exhibits reduced swelling, improved stress crack resistance, and/or greater retention of certain mechanical properties such as hardness, modulus, and strength, upon exposure to alcohol.

The alcohol resistant catheters disclosed herein comprise an alcohol resistant aromatic polycarbonate urethane formed by reacting a polyisocyanate, a polyol, and a chain extender. In such embodiments, the polyisocyanate may be one or more aromatic diisocyanates selected from at least one of 4,4' methylene bis diphenyl diisocyanate (MDI), p-tetramethyl xylene diisocyanate, m-tetramethyl xylene diisocyanate, bitolylene diisocyanate, toluene diisocyanate, p-phenylene diisocyanate, isophorone diisocyanate, 1,5-naphthalene diisocyanate, hexamethylene diisocyanate, methylene-bis cyclohexyl diisocyanate, and isomers and or mixtures thereof.

The alcohol resistant catheters disclosed herein comprise an alcohol resistant aromatic polycarbonate urethane formed by reacting a polyisocyanate, a polyol, and a chain extender wherein the polyol is a polycarbonate polyol. In such embodiments, the polyol may be a polycarbonate polyol having a formula HO-[-R1-O(CO)O-]n-H wherein R1 is an alkyl group of between 4 to 20 methylene units, such that greater than 99% of the R1 groups have the same chemical structure; and n is between 1 and 35. The polyol is a polycarbonate polyol comprising a weight average molecular weight from between 1000 to 3000 g/mol. In embodiments of the present invention, the polyol is a polycarbonate polyol comprising a weight average molecular weight from between 1500 to 2500 g/mol. In yet other such embodiments, the polyol may be a polycarbonate polyol that includes a diol comprising alternating carbonate groups and linear aliphatic chains having from 4 to 20 methylene units. In further such embodiments, the polyol may be a polycarbonate polyol that includes polyhexanediol carbonate. In yet further such embodiments, the polyol may be a polycarbonate polyol that is present in an amount ranging from about 30 wt% to about 70 wt% of the aromatic polycarbonate urethane.

In further embodiments, the alcohol resistant catheters disclosed herein may comprise an alcohol resistant aromatic polycarbonate urethane formed by reacting a polyisocyanate, a polyol, and a chain extender wherein the chain extender is any compound capable of polymerizing with the polyisocyanate such that the chain extender resides in the hard segment of the polyurethane. In some embodiments, the chain extender can be a compound having a molecular weight of less than 500 g/mol. In other embodiments, the chain extender can be selected from at least one of the following: polyols, ethylene glycol, diethylene glycol, neopentyl glycol, 1,3-propane diol, 1,2-propanediol, 2-ethyl-2-(hydroxymethyl)propane-1,3-diol, glycerol, 1,4-butanediol, hydroquinone bis(2-hydroxyethyl)ether, cyclohexane dimethylol, trimethylolpropane, pentanediol, hexanediol, heptanediol, octanediol, nonanediol, decanediol, undecanediol, dodecanediol, and mixtures thereof.

Although a number of aromatic polyurethanes can be utilized in preparing a polymer in accordance with the present disclosure, in some embodiments the chain extender comprises diols with 4 to 14 carbon atoms, 10 to 14 carbon atoms, or 12 carbon atoms, or combinations thereof. In particular embodiments, the chain extender may comprise diols having between 4 and 20 carbon atoms. In certain embodiments, the chain extender may comprise diols having 4, 5, 6, 7, 8, 9, 10, 11, 12, 13, 14, 15, 16, 17, 18, 19 and 20 carbon atoms. In still other embodiments, the chain extender can be present in the aromatic polycarbonate urethane in an amount from about 0.5 wt% to about 45 wt%. In yet other embodiments, the chain extender can be present in the aromatic polycarbonate urethane in an amount from about 0.5 wt% to about 5 wt%. In further embodiments, the chain extender can be present in the aromatic polycarbonate urethane in an amount from about 5 wt% to about 45 wt %.

In further embodiments, the alcohol resistant catheters disclosed herein may comprise an alcohol resistant aromatic polycarbonate urethane having one or more radiopacifiers. Generally, radiopacifiers are dense fillers added to polymers to enable resultant medical devices, including catheter shafts, for instance, to be viewed under radiography when in the body. Radiopacifiers used in medical polymers may include barium sulfate ($BaSO_4$), tungsten metals, bismuth

metals and related species (e.g., bismuthoxide, bismuth oxychloride, bismuth subcarbonate, etc.), platinum, palladium, and gold. The amount of radiopaque filler added to a polymer may vary from about 5 wt% to about 65 wt%. In some instances, the addition of higher amounts of filler and/or more dense fillers may increase the radiopaqueness of the resultant medical catheter shaft, but may also deteriorate the mechanical properties of the material (elongation, tensile strength, burst strength, biocompatibility, modulus, and chemical resistance, for example). Thus, the amount of filler added to a catheter material may be dependent on the particular application requirements of the material. For example, in small diameter, thin walled catheters - which may become difficult to see under radiography - the appropriate amount of filler may depend highly on parameters of the device as well as the expected use of the device.

In the case of thermoplastic polyurethanes, barium sulfate may be used in PICCs. However, barium sulfate may not provide adequate radiopacity in small thin wall catheters without negatively affecting the performance of the catheter. For example, in high pressure small PICC shafts, where the catheter flow rate and burst strength are potential performance requirements, barium sulfate may not provide sufficient radiopacity without compromising the properties of the device. In some applications, bismuth metals and related species show improved radiopacity when compared to a similar weight percent of barium sulfate due to their higher densities. Historically, bismuth metals have not been used in thermoplastic polyurethanes due to polymer degradation, poor UV stability, color, and heat related discoloration.

In some embodiments of the current disclosure, however, a select grade of a bismuth species (e.g., bismuthoxide, etc.) and the present polycarbonate urethanes may not exhibit issues with heat stability, UV stability, and polymer compatibility. Notably, in some applications, such a combination can also provide superior radiopacity without substantial negative impact on the elongation, tensile strength, and chemical resistance of the catheter shaft. In other words, an aromatic thermoplastic urethane with a bismuth species (e.g., bismuthoxide, etc.) radiopacifier may be utilized to produce, for example, a catheter with particular properties for medical catheter shafts as disclosed herein.

Disclosed herein are medical devices, including central venous catheters, that may be formulated to resist the detrimental effects of solvents and chemicals upon overall mechanical properties thus enabling these materials to be used in the presence of alcohol as well as allowing the construction of smaller and more flexible central venous catheters. Specific methods for fabrication of such medical devices may include preparing the aromatic polycarbonate urethanes as recited herein and forming into a desired device.

Also disclosed herein are medical devices that may contain a mixture of aromatic polycarbonate urethanes such as one or more alcohol resistant aromatic polycarbonate urethanes as described herein. Further, the alcohol resistant aromatic polycarbonate urethanes described herein can be used in a number of medical applications. In one embodiment, for example, a medical device or instrument can be coated or manufactured, in whole or in part, with the alcohol resistant aromatic polycarbonate urethanes described herein.

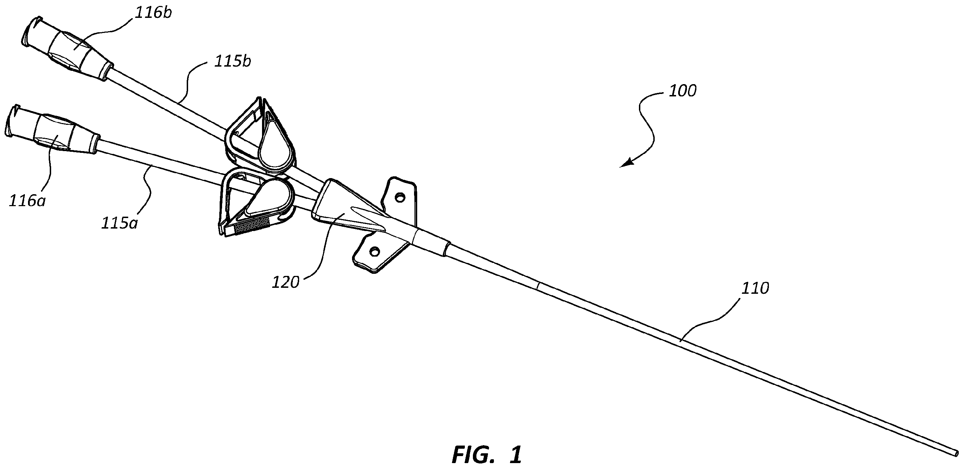

In certain embodiments, a peripherally inserted central line catheter (PICC) may comprise an alcohol resistant aromatic polycarbonate urethane as disclosed herein. Certain such embodiments may include a PICC 100 as shown in Fig. 1. In particular embodiments, a PICC 100 may be constructed from an extruded catheter 110 with one or more lumens that is affixed to corresponding extension leg tubing 115a, 115b via a junction hub 120. The extension leg tubing 115a, 115b may be affixed to luer hubs 116a, 116b that are designed to connect each of the extension legs 115a, 115b to a medical device such as a syringe or tubing. In some embodiments, a PICC 100 comprises an alcohol resistant aromatic polycarbonate catheter shaft with a wall thickness that is from approximately 0.005" (0.013 cm) to approximately 0.021" (0.053 cm) and a length from the junction hub 120 to the distal end of that is from approximately 30 cm to approximately 60 cm. In other embodiments, the PICC 100 comprises an alcohol resistant aromatic polycarbonate catheter shaft with a wall thickness that is from approximately 0.006" (0.015 cm) to approximately 0.015" (0.038 cm). In other embodiments, the wall thickness, outer diameter, and elastic modulus of the material determine the stiffness of the catheter shaft. The wall thickness, lumen area, and elastic modulus determine the use pressure of the catheter during power injection. The wall thickness, diameter, elastic modulus, and tensile strength determine the burst pressure of the catheter during power injection or during a hydraulic load, such as an injection from a syringe by a clinician. Prolonged and direct exposure to a polar solvent such as alcohol or water results in a reduction of elastic modulus. Alcohol causes a greater reduction in elastic modulus than water because it is a stronger polar solvent than water.

The reduction in modulus directly impacts the burst strength of the catheter by the following equation:

$$P_b = CE\frac{t}{r}$$

There $P_b$ is the burst strength of the catheter, C is a constant of proportionality dependent on the rheological properties of the material (typically close to 0.25), E is the modulus of elasticity of the material, t is the wall thickness of the tube, and r is the radius of the tube. Therefore, as the modulus is reduced due to soaking in alcohol or water, the burst strength of the tubing decreases in proportion to that reduction. It is therefore desirable that the material not soften beyond a certain threshold due to alcohol locking.

In certain embodiments, the alcohol resistant catheters disclosed herein comprise an extruded catheter shaft that results in a catheter stiffness of approximately less than 12,000 mN*mm$^2$ when dry and approximately greater than 400 mN*mm$^2$ during or after direct and prolonged exposure to alcohol. In some embodiments, the alcohol resistant catheters disclosed herein can comprise an extruded catheter shaft that results in a catheter stiffness of approximately less than 12,000 mN*mm$^2$ when dry and approximately greater than 2,000 mN*mm$^2$ during or after direct and prolonged exposure to alcohol. In other embodiments, the alcohol resistant catheters disclosed herein may have a stiffness such that the reduction in stiffness between the dry state and alcohol locked state is less than 60%. In other embodiments, the alcohol resistant catheters disclosed herein may have a stiffness such that the reduction in stiffness between the dry state and alcohol locked state is less than 50%. In further embodiments, at least a portion of the alcohol resistant catheters disclosed herein may have a burst pressure that exceeds the use pressure, thereby surviving power injection following an alcohol lock. In still further embodiments, the alcohol resistant catheters disclosed herein may include one or more catheter shafts where the ratio of hydrated stiffness to alcohol lock stiffness is less than 1.5.

In some embodiments, the alcohol resistant catheters disclosed herein comprise an aromatic polycarbonate polyurethane extension leg with a wall thickness that is approximately 0.015" +/- 0.010" (0.038 cm ± 0.025 cm) and a length from the junction hub to the distal end of approximately 4 cm to 10 cm. In particular embodiments, the extension leg is designed to not burst during power injection. In such embodiments, the burst strength of the extension leg may be above 250 psi (1724 kPa) during or after prolonged exposure to alcohol. In other such embodiments, the burst strength of the extension leg may be above 250 psi (1724 kPa) after being clamped during or after prolonged exposure to alcohol. In yet other embodiments, the burst strength of the extension leg can be above 250 psi after being clamped during or after prolonged exposure to alcohol of a period of 45 days. In further embodiments, a wall thickness to diameter ratio of less that 0.25 is desirable so that the extension leg remains flexible.

In particular embodiments, the alcohol resistant catheters disclosed herein comprise an aromatic polycarbonate polyurethane extension junction hub. In such embodiments, the junction hub may connect a particular extension to a particular lumen of the catheter shaft. The catheter can leak if the bond between the junction hub and the catheter shaft or the junction hub and the extension leg tubing is compromised, especially during high pressure events such as during power injection and hydraulic pressure. Additionally, the integrity of the junction hub is reduced as the elastic modulus and the tensile strength of the junction hub polyurethane is reduced such as occurs during prolonged and direct exposure to alcohol.

In other embodiments, a junction hub may be molded with a rigid aromatic polyurethane with a durometer of between Shore 100A and Shore 80D. In specific embodiments, the combination of formulations of polyurethane for the catheter shaft and extension legs, combined with an aromatic polyurethane junction hub and rigid aromatic polyurethane luer connectors may allow for the continued current standard of performance during and after direct and prolonged exposure to alcohol.

Examples

Example 1: Alcohol resistant aromatic polycarbonate urethane polymers

Alcohol resistant aromatic polycarbonate urethane polymer test samples were made according to Table 1 . The test samples were made by standard one shot hand casting method where 4,4' methylene bis diphenyl diisocyanate (MDI), polyol (polyhexamethylene carbonate (PHMC) diol), and a chain extender are weighed into a mixing vessel, stirred for 1 -3 minutes, and poured into pans. The pans were then cured for at least 16 hours at 1 10 °C. The resulting cured sheets were granulated, then compounded on a twin-screw extruder. The resulting pellets were then injection molded into ASTM test plaques. As shown in Table 1 , the Shore A hardness, tensile strength (psi) (KPa), % strain at break, 25% secant modulus (psi) (KPa), and % weight change (% wt chg) were tested for each of the test samples.

Table 1

| Test sample # | 1 | 2 | 3 |
|---|---|---|---|
| Polyol: Description Amount (g) Temperature (°C) | PHMC diol, 2000 g/mol 8785 80 | PHMC diol, 3000 g/mol 41621.3 77 | PHMC diol, 3000 g/mol 10093.8 88 |
| Isocyanate: Description Amount (g) Temperature (°C) | MDI 4250 55 | MDI 17185.8 55 | MDI 3687.6 52 |

(continued)

| Test sample # | 1 | 2 | 3 |
|---|---|---|---|
| Chain Extender:<br>Description<br>Amount (g)<br>Temperature (°C) | <br>1,4-Butanediol<br>1059<br>27 | <br>1,4-Butanediol<br>4730.6<br>27 | <br>1,12-Dodecanediol<br>2242.3<br>88 |
| Radiopacifier:<br>Description<br>Amount (Wt %g) | <br>$BaSO_4$<br>20% | <br>$BaSO_4$<br>30% | <br>$BaSO_4$<br>30% |
| Mechanical Properties: As moulded<br>ShoreA Hardness<br>Tensile Strenght (psi) [kPa]<br>%Strain at Break<br>25% Secant Modulus (psi) [kPa] | <br>[not tested]<br>[not tested]<br>[not tested] | <br>91<br>6687 [46105]<br>405<br>3773 [26013] | <br>93<br>3950 [27234]<br>473<br>3951 [27261] |
| Mechanical Properties: EtOH Aged[a]<br>ShoreA Hardness<br>Tensile Strenght (psi) [kPa]<br>%Strain at Break<br>25% Secant Modulus (psi) [kPa]<br>% Wt Chg | <br>[not tested]<br>[not tested]<br>[not tested]<br>[not tested]<br>[not tested] | <br>81<br>4369 [30123]<br>534<br>1404 [9680]<br>7.5% | <br>83<br>2809 [19367]<br>568<br>1992 [13734]<br>6.3% |
| *a. 46 hours at 37 °C in 70% Ethanol* | | | |

Example 2: Performance of catheters comprising alcohol resistant aromatic polycarbonate urethanes.

Using the aromatic polycarbonate urethanes listed as Test Samples # 1 -3 in Table 1, catheters were fabricated according to the design shown in Fig. 1 using a catheter extrusion process. The test catheters had the following specifications: material = aromatic polycarbonate polyurethane; number of lumens: 2; length = 55 cm; OD = 0.0695 (nominal = 0.068); and lumen area = 0.00088 $in^2$ (nominal= 0.00081) (0.00568 $cm^2$ (nominal = 0.00522 $cm^2$)). The performance of the catheters was compared to a commercially available control catheter comprising Carbothane 3595A. The results are shown in Table 2.

Table 2

| Test Sample Used | Test Sample #1 | Test Sample #2 | Test Sample #3 | Carbothane 3595A |
|---|---|---|---|---|
| Unexposed Catheter Test Results:<br>Stiffness (mN*mm $^2$) | <br>8248 | <br>7498 | <br>[not tested] | <br>4566 |
| | | | | |
| Alcohol Exposed[a] Catheter Test Results:<br>Stiffness (mN*mm $^2$)<br>Burst Pressure (psi) [kPa] | <br><br>5119<br>192 [1324] | <br><br>4402<br>210 [1448] | <br><br>[not tested]<br>225 [1551] | <br><br>1713<br>N/A[b] |
| *a. 46 hours at 37 °C in 70% Ethanol*<br>*b. All Carbothane samples failed during power injection* | | | | |

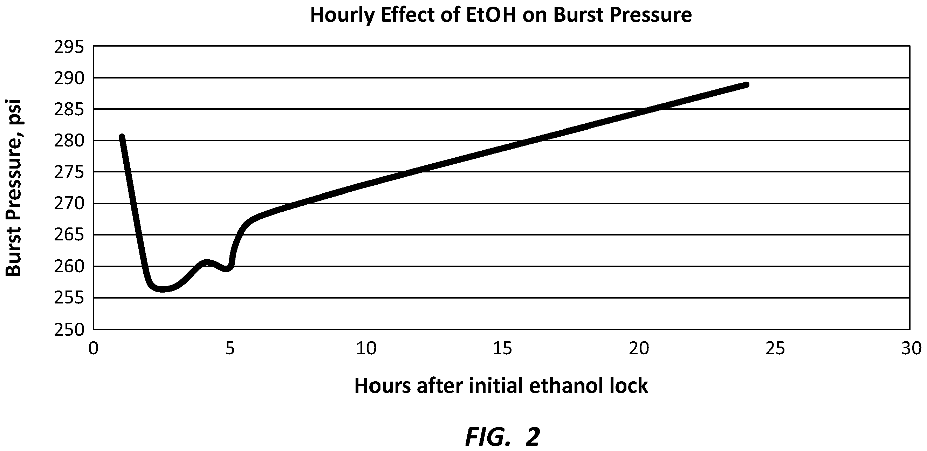

Catheter burst strength was measured before and after direct and prolonged exposure to 70% alcohol for up to 46 hours. With reference to Fig. 2, it was determined that the catheter burst pressure reached a minimum at approximately 2 hours following the ethanol lock and then the stiffness began to increase as the ethanol diluted through the walls of the catheter and into the surrounding bath filled with deionized water. Therefore, just 2 hours following the ethanol lock the catheter reached its approximate minimum burst strength.

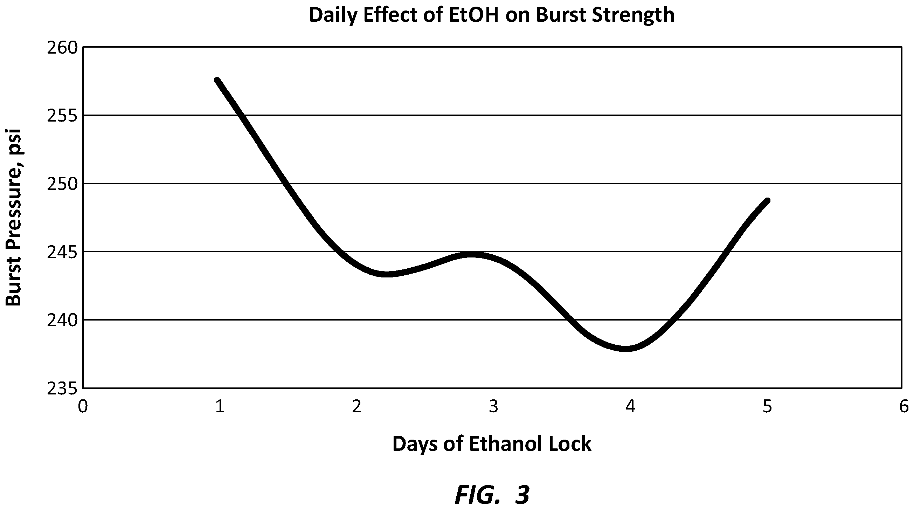

The burst strength was also monitored over the course of several days with the catheter being continuously locked with alcohol, and then relocked with alcohol just 2 hours prior to being burst. As shown Fig. 3, the burst strength showed some degradation but then leveled out over 5 days of exposure.

Example 3: Power injection of alcohol locked catheter samples.

Samples of alcohol resistant catheters were prepared using the aromatic polycarbonate urethane of Test Sample # 3 shown in Table 1.

Group A - 10 Samples of alcohol resistant catheters were locked and clamped 24 hrs a day and power injected for 10 straight days.

Group B - 10 Samples locked and clamped 2 hrs prior to power injection, power injected for 10 straight days.

Group C - 5 Samples locked and clamped 2 hrs prior to power injection, soaked in saline, power injected for 5 straight days.

The results are shown in Table 3. All groups passed power injection testing up to 161 psi (1110 kPa) without bursting and showed no signs of deformation post power injection.

Table 3.

| Sample Group | Average Pressure | Range | Standard Deviation |
|---|---|---|---|
| Group A | 144.81 psi (998.42 kPa) | 132 -161 psi (910 - 1110 kPa) | 5.62 psi (38.75 kPa) |
| Group B | 144.21 psi (994.29 kPa) | 134 - 156 psi (924 - 1076 kPa) | 5.64 psi (38.89 kPa) |
| Group C | 144.82 psi (998.50 kPa) | 137 - 155 psi (945 -1069 kPa) | 4.64 psi (31.99 kPa) |

Example 4: Flexural fatigue.

Aromatic polycarbonate urethanes, listed as Test Samples # 1 -3 in Table 1 , were used to fabricate catheters according to the design shown in Fig. 1 using a catheter extrusion process. The catheters were alcohol locked and were cyclically kinked (representing arm bending at the antecubital fossa) over 200,000 times, and did not leak thereafter when exposed to a constant pressure of 45 psi (310 kPa).

Example 5: Extension leg durability.

The extension legs were extruded from Shore 95A durometer aromatic polycarbonate urethane, with an outside diameter of 0.107" +/- 0.003" (0.212 $\pm$ 0.008 cm) and a wall thickness of: 0.020" +/- 0.002" (0.051 $\pm$ 0.005 cm). The catheter assembly, including the extension legs, was filled with 70% alcohol solution for 24 hours. While the catheter assembly was filled with alcohol the extension leg was clamped 1116 times. The catheter was pressurized to 250 psi (1723 kPa). The extension legs did not burst, and additionally no leaks were observed. Conversely, extension legs constructed from aromatic polyether polyurethane burst during the same test condition.

Example 6: Effect of alcohol on the catheter modulus of elasticity.

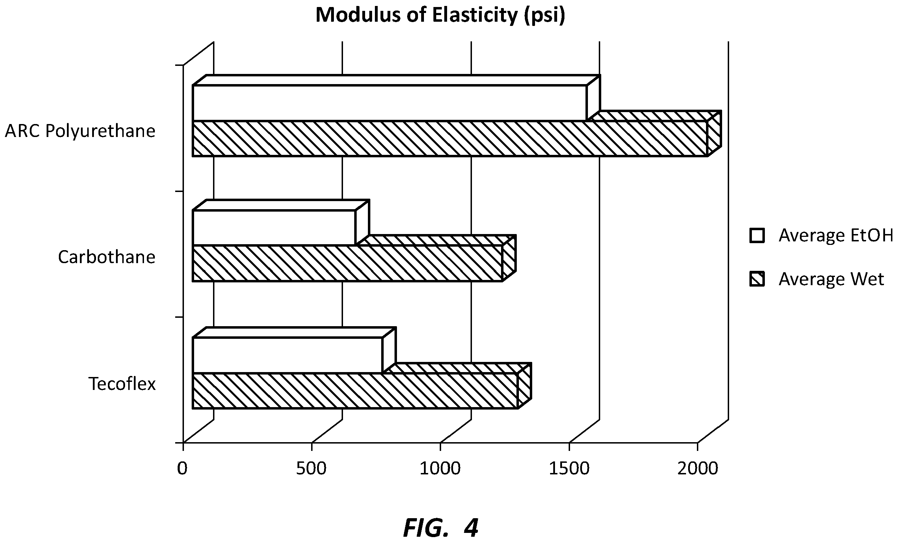

The effect of alcohol on the catheter modulus of elasticity was observed for a catheter comprising the alcohol resistant aromatic polycarbonate urethane of Test Sample # 3 (ARC Polyurethane) from Table 1 and two additional catheters comprising the commercially available Carbothane and Tecoflex. The results shown in Fig. 4 demonstrate that the aromatic alcohol resistant aromatic polycarbonate urethane of Test Sample # 3 shows a higher modulus of elasticity than the commercial formulations for both water and ethanol. "Wet" refers to soaking in 37°C water for a minimum of 2 hours. "EtOH" refers to locking the device with a 70% ethanol solution, then submerging the catheter shaft in a 37°C water bath for 2 hours.

Example 7: Effect of alcohol on catheter tensile strength.

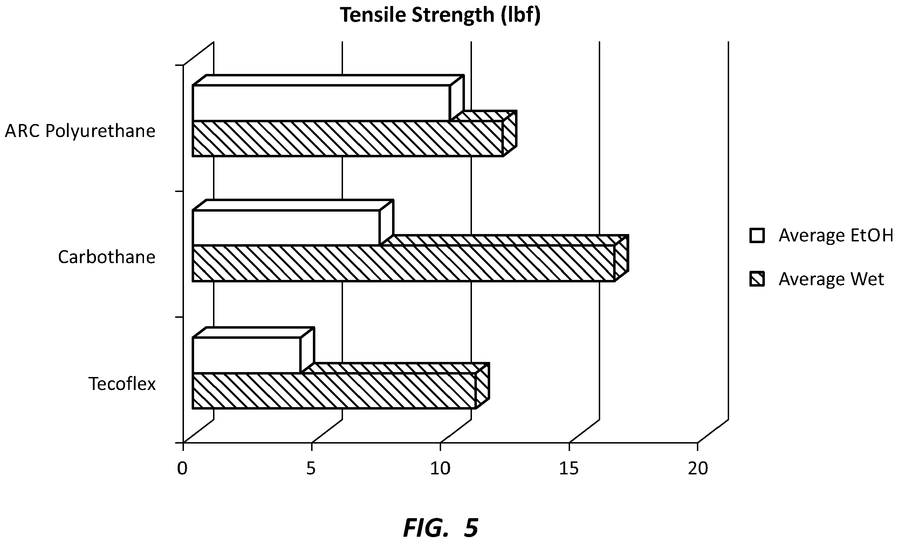

The effect of alcohol on the catheter tensile strength in pounds of force (lbf) (1 lbf=4,45 newtons (N)) was tested for a catheter comprising the alcohol resistant aromatic polycarbonate urethane of Test Sample # 3 (ARC Polyurethane) from Table 1 and two additional catheters comprising the commercially available Carbothane and Tecoflex. The results are shown in Fig. 5 and reveal that the alcohol resistant aromatic polycarbonate urethane of Test Sample # 3 has a higher average tensile strength after alcohol lock than the commercial formulations Carbothane and Tecoflex.

Example 8: Power injection results of catheters after ethanol lock.

Table 4 shows a comparison of power injection results of different catheters manufactured with various polyurethanes and the Test Sample # 3, following a 2 hour 70% ethanol lock. Power injection was performed with 37c visipaque 320 contrast (11.8 cP), using the flow rates as specified.

Table 4

| Sample Name | Polyurethane Shaft Material | Configuration | Length | Flow Rate | Result |
|---|---|---|---|---|---|
| Medcomp Pro-PICC CT | Aromatic Polyether | 5F DL | 55 cm | 5.0 mL/s | *Burst* |
| Navilyst Xcela PASV | Aliphatic Polycarbonate | 5F DL | 55 cm | 4.0 mL/s | *Burst* |
| AngioDynamics Morpheus CT | Aliphatic Polycarbonate | 5F DL | 65 cm | 4.0 mL/s | *Burst* |
| Arrow Pressure Injectable PICC | Aromatic Polyether | 5F DL | 50 cm | 4.0 mL/s | *Burst* |
| Cook Spectrum Turbo-JeCT | Aliphatic Polyether | 5F DL | 55 cm | 5.0 mL/s | *Burst* |
| BARD PowerPICC | Aliphatic Polyether | 5F DL | 55 cm | 5.0 mL/s | *Burst* |
| Test Sample #3 | Aromatic Polycarbonate | 5F DL | 55 cm | 5.0 mL/s | *Pass* |

**Claims**

1. An alcohol resistant catheter, comprising:

    a catheter shaft with at least one lumen and comprising an alcohol resistant aromatic polycarbonate urethane;
    an aromatic polycarbonate urethane junction hub; and
    at least one aromatic polycarbonate urethane extension tube;
    wherein the alcohol resistant aromatic polycarbonate urethane is formed by reacting at least the following: polyisocyanate, polycarbonate polyol, and a chain extender; and
    wherein the polycarbonate polyol has a weight average molecular weight of between 1000 and 3000 g/mol.

2. The alcohol resistant catheter of claim 1 wherein the junction hub further comprises a soft outer molded junction surrounding a hard inner junction.

3. The alcohol resistant catheter of any one of claims 1 - 2, wherein the aromatic polycarbonate urethane of the extension tube is alcohol resistant.

4. The alcohol resistant catheter of any one of claims 1 - 3, further comprising at least one alcohol resistant luer connector comprising an aromatic polycarbonate urethane.

5. The alcohol resistant catheter of any one of claims 1 - 4, wherein the extension tube comprises a clampable, alcohol lockable extension tube.

6. The alcohol resistant catheter of any one of claims 1 - 5 , wherein the chain extender is a diol having between 4 and 20 carbon atoms.

7. The alcohol resistant catheter of any one of claims 1 - 6 wherein the polycarbonate polyol has the following formula: HO-[-R1-O(CO)O-]$_n$-H wherein R1 is an alkyl group of between 4 to 20 methylene units, such that greater than 99% of the R1 groups have the same chemical structure; and n is between 1 and 35.

8. The alcohol resistant catheter of any one of claims 1-7, wherein the polycarbonate polyol is a diol comprising alternating carbonate groups and linear aliphatic chains having from 4 to 20 methylene units.

9. The alcohol resistant catheter of any one of claims 1-8, wherein the aromatic polycarbonate urethane further comprises a radiopacifier.

10. The alcohol resistant catheter of any one of claims 1-9, wherein the polycarbonate polyol is present in an amount ranging from 30 wt% to 70 wt% of the aromatic polycarbonate urethane.

11. The alcohol resistant catheter of any one of claims 1-10, wherein the polycarbonate polyol is a polyhexanediol carbonate.

12. The alcohol resistant catheter of any one of claims 1-11, wherein the polyisocyanate is a diisocyanate selected from at least one of the following: 4,4' methylene bis diphenyl diisocyanate, hexamethylene diisocyanate, p-tetramethyl xylene diisocyanate, m-tetramethyl xylene diisocyanate, bitolylene diisocyanate, toluene diisocyanate, methylene-bis cyclohexyl diisocyanate, p-phenylene diisocyanate, isophorone diisocyanate, 1,5-naphthalene diisocyanate, and isomers and/or mixtures thereof.

**Patentansprüche**

1. Alkoholresistenter Katheter, umfassend:

    einen Katheterschaft mit zumindest einem Lumen und umfassend ein alkoholresistentes aromatisches Polycarbonat-Urethan;
    eine Kanaltrennung aus aromatischem Polycarbonat-Urethan; und
    zumindest einen Anschlussschlauch aus aromatischem Polycarbonat-Urethan;
    wobei das alkoholresistente aromatische Polycarbonat-Urethan durch Reaktion aus zumindest dem Folgenden erzeugt worden ist: Polyisocyanat, Polycarbonatpolyol und einem Kettenverlängerer; und
    wobei das Polycarbonatpolyol ein gewichtsgemitteltes Molekulargewicht von zwischen 1000 und 3000 g/mol hat.

2. Alkoholresistenter Katheter nach Anspruch 1, wobei die Kanaltrennung weiterhin eine weiche äußere gegossene Kanaltrennung umfasst, die eine harte innere Kanaltrennung umgibt.

3. Alkoholresistenter Katheter nach einem der Ansprüche 1-2, wobei das aromatische Polycarbonat-Urethan des Anschlussschlauchs alkoholresistent ist.

4. Alkoholresistenter Katheter nach einem der Ansprüche 1-3, weiterhin umfassend zumindest einen alkoholresistenten Luer-Verbinder, der ein aromatisches Polycarbonat-Urethan umfasst.

5. Alkoholresistenter Katheter nach einem der Ansprüche 1-4, wobei der Anschlussschlauch einen abklemmbaren alkoholblockierbaren Anschlussschlauch umfasst.

6. Alkoholresistenter Katheter nach einem der Ansprüche 1-5, wobei der Kettenverlängerer ein Diol ist, das zwischen 4 und 20 Kohlenstoffatome aufweist.

7. Alkoholresistenter Katheter nach einem der Ansprüche 1-6, wobei das Polycarbonatpolyol die folgende Formel hat: HO-[-R1-O(CO)O-]$_n$-H wobei R1 eine Alkylgruppe von zwischen 4 bis 20 Methyleneinheiten ist derart, dass mehr als 99% der R1-Gruppen dieselbe chemische Struktur aufweisen; und wobei n zwischen 1 und 35 ist.

8. Alkoholresistenter Katheter nach einem der Ansprüche 1-7, wobei das Polycarbonatpolyol ein Diol ist, das alternierende Carbonatgruppen und lineare aliphatische Ketten umfasst, die von 4 bis 20 Methyleneinheiten aufweisen.

9. Alkoholresistenter Katheter nach einem der Ansprüche 1-8, wobei das aromatische Polycarbonat-Urethan weiterhin ein röntgendichtes Material umfasst.

**10.** Alkoholresistenter Katheter nach einem der Ansprüche 1-9, wobei das Polycarbonatpolyol in einer Menge von 30% w/w bis 70% w/w des aromatischen Polycarbonat-Urethans vorhanden ist.

**11.** Alkoholresistenter Katheter nach einem der Ansprüche 1-10, wobei das Polycarbonatpolyol ein Polyhexandiolcarbonat ist.

**12.** Alkoholresistenter Katheter nach einem der Ansprüche 1-11, wobei das Polyisocyanat ein Diisocyanat ist, das als zumindest eins der Folgenden ausgewählt ist: 4,4'-Methylenbisdiphenyldiisocyanat, Hexamethylendiisocyanat, p-Tetramethylxyloldiisocyanat, m-Tetramethylxyloldiisocyanat, Bitolylendiisocyanate, Toluoldiisocyanat, Methylenbiscyclohexyldiisocyanat, p-Phenylendiisocyanat, Isophorondiisocyanat, 1,5-Naphthalendiisocyanat, und Isomere und/oder Mischungen derselben.

## Revendications

**1.** Cathéter résistant à l'alcool, comprenant :

une tige de cathéter avec au moins une lumière et comprenant un polycarbonate uréthane aromatique résistant à l'alcool ;
un point de jonction en polycarbonate uréthane aromatique ; et
au moins un tube d'extension en polycarbonate uréthane aromatique ;
dans lequel le polycarbonate uréthane aromatique résistant à l'alcool est formé par la réaction d'au moins les suivants : un polyisocyanate, un polycarbonate polyol, et un extenseur de chaîne ; et
dans lequel le polycarbonate polyol a un poids moléculaire moyen en poids situé entre 1 000 et 3 000 g/mol.

**2.** Cathéter résistant à l'alcool selon la revendication 1 dans lequel le point de jonction comprend en outre une jonction moulée externe souple entourant une jonction interne dure.

**3.** Cathéter résistant à l'alcool selon l'une quelconque des revendications 1 à 2, dans lequel le polycarbonate uréthane aromatique du tube d'extension est résistant à l'alcool.

**4.** Cathéter résistant à l'alcool selon l'une quelconque des revendications 1 à 3, comprenant en outre au moins un connecteur Luer résistant à l'alcool comprenant un polycarbonate uréthane aromatique.

**5.** Cathéter résistant à l'alcool selon l'une quelconque des revendications 1 à 4, dans lequel le tube d'extension comprend un tube d'extension à blocage d'alcool, serrable.

**6.** Cathéter résistant à l'alcool selon l'une quelconque des revendications 1 à 5, dans lequel l'extenseur de chaîne est un diol ayant entre 4 et 20 atomes de carbone.

**7.** Cathéter résistant à l'alcool selon l'une quelconque des revendications 1 à 6, dans lequel le polycarbonate polyol a la formule suivante :
HO-[-R1-O(CO)O-]$_n$-H dans lequel R1 est un groupe alkyle ayant entre 4 à 20 unités de méthylène, de sorte que plus de 99 % des groupes R1 ont la même structure chimique ; et n est entre 1 et 35.

**8.** Cathéter résistant à l'alcool selon l'une quelconque des revendications 1 à 7, dans lequel le polycarbonate polyol est un diol comprenant des groupes carbonate alternés et des chaînes aliphatiques linéaires ayant de 4 à 20 unités de méthylène.

**9.** Cathéter résistant à l'alcool selon l'une quelconque des revendications 1 à 8, dans lequel le polycarbonate uréthane aromatique comprend en outre un radio-opacifiant.

**10.** Cathéter résistant à l'alcool selon l'une quelconque des revendications 1 à 9, dans lequel le polycarbonate polyol est présent dans une quantité allant de 30 % en poids à 70 % en poids du polycarbonate uréthane aromatique.

**11.** Cathéter résistant à l'alcool selon l'une quelconque des revendications 1 à 10, dans lequel le polycarbonate polyol est un carbonate de polyhexanediol.

12. Cathéter résistant à l'alcool selon l'une quelconque des revendications 1 à 11, dans lequel le polyisocyanate est un diisocyanate sélectionné parmi au moins l'un des suivants : le 4,4'-bisdiphényldiisocyanate de méthylène, le diisocyanate d'hexaméthylène, le diisocyanate de p-tétraméthylxylène, le diisocyanate de m-tétraméthylxylène, le diisocyanate de bitolylène, le diisocyanate de toluène, le diisocyanate de méthylène-bis cyclohexyle, le diisocyanate de p-phénylène, le diisocyanate d'isophorone, le diisocyanate de 1,5-naphtalène, et des isomères et/ou des mélanges de ceux-ci.

**FIG. 1**

EP 2 968 682 B1

Hourly Effect of EtOH on Burst Pressure

Burst Pressure, psi

Hours after initial ethanol lock

*FIG. 2*

EP 2 968 682 B1

FIG. 3

**Modulus of Elasticity (psi)**

FIG. 4

EP 2 968 682 B1

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2248542 A
- US 2007007437 A
- WO 2008024514 A